# EUROPÄISCHE PATENTSCHRIFT

(11) **EP 1 915 464 B1**
(45) Veröffentlichungstag und Bekanntmachung des Hinweises auf die Patenterteilung: **17.06.2009**
(21) Anmeldenummer: 06791587.6
(22) Anmeldetag: 17.08.2006
(51) Int. Cl.: C12Q 1/68

(54) **VERFAHREN ZUR MARKIERUNG UND ANALYSE VON NUKLEINSÄUREN**
METHOD FOR LABELLING AND ANALYSING NUCLEIC ACIDS
PROCEDE DE MARQUAGE ET D'ANALYSE D'ACIDES NUCLEIQUES

(30) Priorität: 19.08.2005 DE 102005039726
(43) Veröffentlichungstag der Anmeldung: 30.04.2008
(73) Patentinhaber: Universität Rostock, 18055 Rostock (DE)
(72) Erfinder: FLECHSIG, Gerd-Uwe, 17166 Teterow (DE); RESKE, Thomas, 18057 Rostock (DE)
(74) Vertreter: UEXKÜLL & STOLBERG
(86) Internationale Anmeldenummer: PCT/EP2006/008131
(87) Internationale Veröffentlichungsnummer: WO 2007/020093

(56) Entgegenhaltungen:
- EP-A1- 0 435 470
- WO-A-20/05005952
- UMEK R M ET AL: "Electronic detection of nucleic acids: A versatile platform for molecular diagnostics" JOURNAL OF MOLECULAR DIAGNOSTICS 2001 UNITED STATES, Bd. 3, Nr. 2, 2001, Seiten 74-84, XP002425405 ISSN: 1525-1578
- PALECEK E ET AL: "DETECTING DNA HYBRIDIZATION AND DAMAGE" ANALYTICAL CHEMISTRY, AMERICAN CHEMICAL SOCIETY. COLUMBUS, US, Bd. 73, Nr. 3, 1. Februar 2001 (2001-02-01), Seiten 74A-83A, XP009080149 ISSN: 0003-2700
- LUCARELLI FAUSTO ET AL: "Carbon and gold electrodes as electrochemical transducers for DNA hybridisation sensors." BIOSENSORS & BIOELECTRONICS, Bd. 19, Nr. 6, 15. Januar 2004 (2004-01-15), Seiten 515-530, XP002425406 ISSN: 0956-5663
- FOJTA MIROSLAV ET AL: "Two-surface strategy in electrochemical DNA hybridization assays: Detection of osmium-labeled target DNA at carbon electrodes." ELECTROANALYSIS, Bd. 15, Nr. 5-6, April 2003 (2003-04), Seiten 431-440, XP002425407 ISSN: 1040-0397 in der Anmeldung erwähnt
- FLECHSIG GERD-UWE ET AL: "DNA hybridization detection at heated electrodes." LANGMUIR : THE ACS JOURNAL OF SURFACES AND COLLOIDS 16 AUG 2005, Bd. 21, Nr. 17, 16. August 2005 (2005-08-16), Seiten 7848-7853, XP002425408 ISSN: 0743-7463
- FLECHSIG GERD-UWE ET AL: "Electrochemical detection of DNA hybridization by means of osmium tetroxide complexes and protective oligonucleotides." ANALYTICAL CHEMISTRY 1 MAR 2007, Bd. 79, Nr. 5, 1. März 2007 (2007-03-01), Seiten 2125-2130, XP002425409 ISSN: 0003-2700

## Beschreibung

Um eine elektrochemische Detektierung von Hybridisierungsereignissen der Nukleinsäuren zu ermöglichen, werden häufig redoxaktive Moleküle eingesetzt, die entweder an den Target- oder an einen Reporterstrang kovalent gebunden sind, sich elektrostatisch an die Phosphatgruppen der Nukleinsäuren binden oder sich als Interkalatoren in den Doppelstrang einlagern. Die kovalent fixierten so genannten Redoxmarker werden bei der Herstellung der Target,- oder Reporterstränge angebunden.

Alternativ kann Osmium(VIII)oxid in Form von Komplexen mit 2,2'-Bipyridin, [Os04(bipy)], eingesetzt werden. Diese Komplexverbindungen reagieren spezifisch mit den Pyrimidinbasen Thymin, Uracil und, in weitaus geringerem Maße, mit Cytosin, wobei die Doppelbindung im Pyrimidinring angegriffen wird. Diese Reaktion findet in einem intakten Doppelstrang nicht statt. Andererseits können Einzelstränge, deren Pyrimidinbasen mit Osmiumkomplexen reagiert haben, keine Doppelstränge mehr bilden. Diese Zusammenhänge wurden seit Anfang der 80er Jahre von Palecek, Jelen und Foita untersucht und in Form zahlreicher Beispiele zur Detektion der Hybridisierung verwendet. Es wird auf folgende Publikationen verwiesen, die hiermit ausdrücklich in Bezug genommen werden: M. Foita et al. J. Am. Chem. Soc. 126 (2004) 6532; P. Kostecka et al. Bioelectrochemistry 63 (2004) 245; M. Foita et al. Electroanalysis 15 (2003) 431.

Die Modifikation von DNA mittels Osmium(VIII)-Komplexen kann sehr einfach durch Zugeben des Osmiumreagenzes zur Analysenlösung erfolgen. Nach der Modifizierung wird das überschüssige Reagenz durch Dialyse in handelsüblichen einfachen Dialysegefäßen abgetrennt. Dazu wird optimalerweise die Hybridisierung von Target und Sonde an unterschiedlichen Oberflächen durchgeführt.

Besonders geeignet sind so genannte Magnetic Beads, welche oberflächlich immobilisierte Sondenstränge tragen und mittels Magnetfeld von der Analyselösung (und damit von nicht komplementären Strängen und Analysereagenzien) getrennt werden können. Nach diesem Prinzip könnte man beliebige Nukleinsäurestränge markieren. Nachteilig ist aber, dass unterschiedliche Reaktionsflächen verwendet werden müssen, um sowohl Hybridisierung als auch elektrochemische Detektierung zu verwirklichen. Das Problem, dass osmiummodifizierte Nukleinsäureeinzelstränge nicht mehr zur Doppelstrangbildung fähig sind und andererseits intakte Doppelstränge nicht mit dem [OsO4(bipy)] reagieren, ist bisher nicht zufrieden stellend gelöst. Wünschenswert wäre die Markierung von PCR-Produkten oder nativen Nukleinsäureproben mit Osmiumverbindungen, ohne dass die Fähigkeit zur Hybridisierung verloren ginge. Die große Zahl der an die Targetstränge gebundenen Osmiumeinheiten würde eine sehr hohe Empfindlichkeit bei der anschließenden elektrochemischen Analyse bewirken. Nachteilig ist auch die Toxizität der Osmiumverbindungen, so dass es wünschenswert wäre, sie durch weniger giftige Substanzen zu ersetzen, welche in analoger Weise mit Nukleinsäuren reagieren.

Aufgabe der Erfindung ist es daher, ein Verfahren bereitzustellen, das die schnelle und einfache Markierung beliebiger Nukleinsäurestränge sowie direkt anschließend die elektrochemische Detektierung an einer Arbeitselektrode ermöglicht.

### Beschreibung der Erfindung

Gelöst wird diese Aufgabe durch ein Verfahren, bei dem man die nachzuweisende Nukleinsäure in Form einzelsträngiger Nukleinsäurestränge, die partiell (d.h. über einen Teil der Gesamtlänge der Nukleinsäurestränge) mit Schutzsträngen hybridisiert sind, mit Substanzen in Kontakt bringt, die spezifisch mit Nukleobasen (Pyrimidinringen) der einzelsträngigen Abschnitte der Nukleinsäurestränge reagieren und die anschließend in dem Fachmann bekannten elektroanalytischen Verfahren eine reversible Redoxreaktion eingehen können.

Dabei verwendet man als Redoxmarker die Osmium (VIII)-Verbindungen [OsO₄(bipy)] oder [OsO₄(py)₂]. Erfindungsgemäß bevorzugt ist der Komplex von Osmiumtetroxid mit 2,2'-Bipyridin, [OsO₄(bipy)]

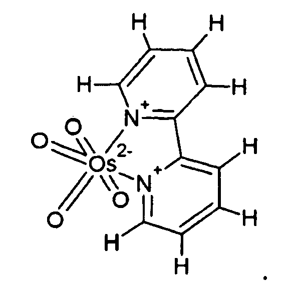

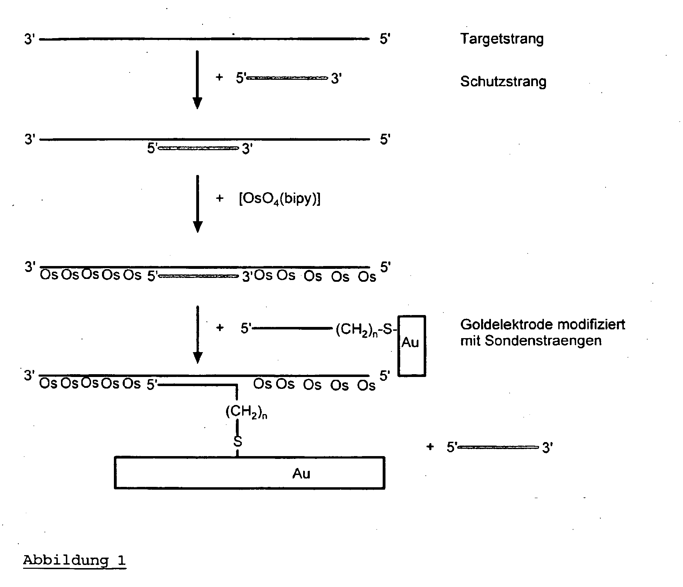

Das erfindungsgemäße Verfahren ist an einem konkreten Beispiel in Abbildung 1 schematisch dargestellt.

Die Erfindung betrifft insbesondere ein Verfahren zum Nachweis von Nukleinsäuresträngen durch Markierung mit Redoxmarkern und elektrochemischer Detektierung von Hybridisierungsereignissen, bei dem man
a) einzelsträngige Nukleinsäurestränge (Targetstränge) mit einem oder mehreren Nukleinsäuresträngen (Schutzsträngen A) hybridisiert, die kürzer als die Targetstränge sind, um partielle Doppelstrangabschnitte auszubilden, man
b) die verbliebenen Einzelstrangabschnitte der Targetstränge durch Reaktion mit den Redoxmarkern [OsO₄(bipy)] oder [OsO₄(py)₂] markiert, die eine elektroanalytisch nutzbare (reversible) Redoxreaktion an Arbeitselektroden (insbesondere an Metall-, Kohle-, Polymer-, Halbleiter-, Indiumzinnoxid-Arbeitselektroden), auf welchen die Sondenstränge immobilisiert sind, ermöglichen, man
c) die so markierten Nukleinsäurestränge an der Oberfläche einer Elektrode mit dort immobilisierten Sondensträngen unter Verdrängung der Schutzstränge A hybridisiert und man
d) die an den Sondensträngen hybridisierten Nukleinsäurestränge anschließend elektroanalytisch nachweist.

Unter "partielle Doppelstrangabschnitte" versteht man vorliegend, dass die einzelsträngigen Targetstränge nur über einen Teil ihrer Gesamtlänge mit Schutzsträngen A) hybridisieren und sich dadurch doppelsträngige Abschnitte bilden, d.h., die Länge der Schutzstränge A) so gewählt wird, dass noch ausreichend lange einzelsträngige Abschnitte der Targetsequenz für die nachfolgende Reaktion mit den Redoxmarkern zur Verfügung stehen. Diese verbleibenden einzelsträngigen Abschnitte der Targetsequenz müssen mindestens eine Thyminbase enthalten. Je mehr Thyminbasen im Einzelstrangabschnitt vorliegen, desto mehr können im anschließenden Schritt mit dem Redoxmarker markiert werden und desto höher werden zum Schluss die elektroanalytischen Signale und damit die Empfindlichkeit des ganzen Verfahrens sein.

Die Hybridisierung erfolgt mit Schutzsträngen A), die kürzer als die Targetstränge sind. Die Länge der Schutzstränge A) muss dabei ausreichend sein, um eine ausreichend feste Doppelstrangbildung zu ermöglichen. Andererseits erfolgt in Schritt c) eine Hybridisierung mit Sondensträngen, die an der Oberfläche einer Elektrode immobilisiert sind. Da diese Hybridisierung unter Verdrängung der Schutzstränge A erfolgt, ist folgendes zu beachten: Die Sondenstränge sollten genauso lang sein wie die Schutzstränge oder nur geringfügig länger. Es ist von Vorteil, wenn im Target-Schutzstrang-Duplex Fehlpaarungen auftreten. Dieser Vorteil kommt insbesondere zum Tragen, wenn die Sondenstränge etwas kürzer als die Schutzstränge sind. Die Fehlpaarungen dürfen nicht an Stellen des Targetstranges auftreten, an denen Thymin-Basen sitzen, da diese sonst markiert werden könnten, was eine spätere Hybridisierung mit den Sondensträngen beeinflussen würde.

So wird gewährleistet, dass der Targetstrang-Schutzstrang-Duplex nicht stabiler als der Targetstrang-Sondenstrang-Duplex ist, und dass ein Tausch der Schutzstränge gegen die Sondenstränge stattfindet.

Gemäß einer besonderen Ausführungsform führt man bei dem o.g. Verfahren Schritt c), d.h. die Verdrängung der Schutzstränge durch die Sondenstränge, bei einer Temperatur durch, die optimal für die thermisch stringente Hybridisierung von Sonden- und Targetsträngen ist. Dadurch wird einerseits der Strangaustausch an der Elektrodenoberfläche beschleunigt und andererseits die Anbindung von nicht hundertprozentig komplementären Strängen an die Sondenstränge erschwert. Die optimale Temperatur liegt unterhalb der sogenannten Schmelztemperatur. Bei letzterer liegen die beiden komplementären Stränge zu 50% als Doppelstrang vor. Zur Ermittlung der optimalen Temperatur eines jeden Sondenstranges bedient man sich der Schmelzkurvenanalyse. Dabei wird ein Analysensignal, welches vom Hybridisierungszustand abhängt, gegen die Temperatur aufgetragen. In homogener Lösung werden dazu üblicherweise die UV-Absorption oder die Fluoreszenz der DNA gemessen.

Der elektroanalytische Nachweis der Targetstränge, welche an die immobilisierten Sondenstränge angebunden sind, erfolgt im Rahmen des erfindungsgemäßen Verfahrens vorzugsweise mittels Chronopotentiometrie, Coulometrie, Amperometrie oder Voltammetrie, vorzugsweise mittels Square-Wave-Voltammetrie (SWV), Wechselstromvoltammetrie (ACV) oder cyclischer Voltammetrie (CV). Als Elektrode verwendet man Metall-, Kohle-, Polymer- oder Halbleiterelektroden, vorzugsweise Draht- oder Schichtelektroden, welche aus Gold, Kupfer, Bismut, Quecksilber, Silber, Blei, Zinn bzw. deren Legierungen bestehen. Bevorzugt kommen Elektroden zum Einsatz, welche mittels elektrischen Stromes direkt oder mittels eines Widerstandsheizers indirekt heizbar sind.

Die Immobilisierung der Sondenstränge auf der Oberfläche der Elektroden erfolgt nach dem Fachmann bekannten Verfahren.

Dabei ist es erfindungsgemäß bevorzugt, die Sondenstränge auf Metallelektroden vorzugsweise mittels Thiolgruppen zu chemisorbieren (vgl. z.B. Steel, B.A.; Herne, T.M.; Tarlov, M.J. Anal. Chem. 1998, 70, 4670-4677).

In einer besonderen Ausführung des erfindungsgemäßen Verfahrens weisen die Doppelstrangabschnitte aus den Schutzsträngen A und den Targetsträngen eine oder mehrere Fehlpaarungen auf, zur leichteren Verdrängung durch die besser passenden, d.h. weniger Fehlpaarungen aufweisenden, Sondenstränge.

Vorteilhafterweise verwendet man in dem Verfahren als Redoxmarker [OsO4(bipy)].

Gemäß einer Ausführungsform der Erfindung führt man das Verfahren durch, indem man vor Duchführung des Schrittes *c*), also nach der Markierungsreaktion zwischen partiell geschützten Targetsträngen und Redoxmarkern, überschüssige Redoxmarkermoleküle durch Dialyse aus der Analysenlösung entfernt.

Gemäß einer weiteren Ausführungsform liegen die Sondenstränge in Schritt c) in hybridisierter Form vor, d.h. sie sind mit einem oder mehreren Nukleinsäuresträngen (Schutzsträngen B) hybridisiert. In diesem Fall hybridisiert man in Schritt c) die markierten Nukleinsäurestränge an der Oberfläche einer Elektrode mit den dort immobilisierten Sondensträngen unter Verdrängung der Schutzstränge A und Schutzstränge B.

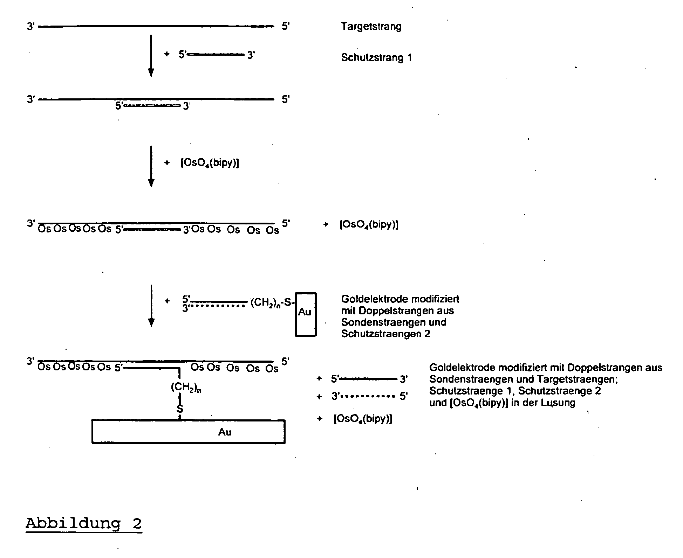

Die vorstehend beschriebene Variante des erfindungsgemäßen Verfahrens ist an einem konkreten Beispiel in Abbildung 2 schematisch dargestellt.

Gemäß einer weiteren Verfahrensvariante verbleiben die überschüssigen Redoxmarker nach Schritt b) in der Analysenlösung, und man trennt die elektrochemischen Signale der an der Oberfläche der Elektrode gebundenen Redoxmarker von den Signalen des in Lösung befindlichen Redoxmarker bei der Detektierung durch geeignete elektrochemische Analyseverfahren voneinander. Bei dieser Verfahrensvariante ist die Chronocoulometrie als elektrochemisches Analyseverfahren bevorzugt. Die Chronocoulometrie gestattet die Unterscheidung zwischen elektrochemischen Diffusionsströmen, welche von der Umsetzung gelöster Substanzen stammen und elektrochemischen Strömen, die von oberflächlich fixierten Substanzen verursacht werden. Auf diese Weise lässt sich z.B. die Menge immobilisierter DNA auf Goldelektroden bestimmen, indem man Rutheniumhexamminchlorid zur Lösung dazu setzt. Dieses bindet sich an die Phosphatgruppen der DNA-Stränge (vgl. z.B. Steel, B.A.; Herne, T.M.; Tarlov, M.J. Anal. Chem. 1998, 70, 4670-4677).

Das erfindungsgemäße Verfahren hat den Vorteil, dass es leicht durchführbar ist und die schnelle und einfache Markierung beliebiger Nukleinsäurestränge ermöglicht, die sich anschließend direkt elektrochemisch detektieren lassen.

Der Vorteil einer Markierung von Nukleinsäuren mit Hilfe von Osmiumtetroxidkomplexen besteht in der einfachen Durchführung. Man muss lediglich die DNA-haltige Lösung mit dem Komplex, z.B. [OsO4(bipy)] versetzen, dann reagiert diese Verbindung spontan mit allen Thymin-Basen in der Lösung, die nicht in DNA-Doppelsträngen, d.h. in DNA-Einzelsträngen oder in einzelsträngigen DNA-Abschnitten vorliegen. Auch andere Pyrimidinbasen werden auf diese Weise angegriffen. Cytosin reagiert allerdings etwa 10-mal langsamer als Thymin. In der RNA tritt die Pyrimidinbase Uracil an die Stelle des Thymins. Sie unterscheidet sich vom Thymin lediglich durch eine fehlende Methylgruppe.

Der zweite Vorteil ist die hohe Empfindlichkeit, die erreicht wird, wenn der Targetstrang viele Thymin- bzw. Uracilbasen enthält. Denn der größte Teil hiervon wird stets im Einzelstrangabschnitt auftreten und damit vom [OsO4(bipy)] angegriffen werden. Im Schnitt kann man bei einem PCR-Produkt mit 50 bis 200 Thyminbasen pro Strang rechnen. mRNA kann über 500 Uracilbasen enthalten. Dies führt zu einem mehr als hundertfach verstärkten Signal verglichen mit herkömmlichen kovalent gebundenen Redoxmarkern wie z.B. Ferrocen, weil pro Targetstrang über 100 Elektronen umgesetzt werden. Eine solche hochempfindliche, selektive und direkte Bestimmung von mRNA kommt ohne einen für gewöhnlich vorangehenden PCR-Schritt aus und ermöglicht so eine schnelle und einfache Analyse der Genexpression von Zellen.

Die erfindungsgemäße Verfahrensmaßnahme, einen oder mehrere Abschnitte der Targetstränge mit einem dazu passenden Schutzstrang zu hybridisieren, hat den Vorteil, dass der oder die so gebildete(n) Doppelstrangabschnitt(e) gegenüber [OsO₄(bipy)] unempfindlich ist. Im Hybridisierungsschritt mit der immobilisierten Sonde wird der Schutzstrang am Targetstrang durch den Sondenstrang ersetzt. Im Ergebnis erhält man einen an der Elektrodenoberfläche gebundenen Targetstrang mit sehr vielen Osmium-markierten Pyrimidinbasen. Hier dargelegt, aber nicht Gegenstand der Erfindung ist, dass Organische Moleküle das Verhalten von [OsO₄(bipy)] imitieren können.

Dazu enthalten sie eine 1,3-Dien-Komponente, um mit der Doppelbindung der Pyrimidinbasn im Sinne einer Diels-Alder-Reaktion zu reagieren, wobei sich ein neuer Ring bildet. Außerdem enthalten diese Moleküle eine Komponente, die in der Lage ist, elektrochemisch reversibel zu reagieren. Dazu gehören Chinone, Naphthachinone, 1,4-Diaminonaphthaline, Diaminobenzole, Anthrachinone, Phenothiazone, Tetrathiafulvalen. Beide Komponenten sind derart verbunden, dass keine Beeinträchtigung ihrer Funktion durch mesomere Effekte gegeben ist. Sterisch sind diese Moleküle so ausgelegt, dass sie den Osmiumkomplexen ähneln und analog zu den Osmiumverbindungen [OsO₄(bipy)] und [OsO₄(py)₂] nur mit dem Nukleinsäureeinzelstrang aber nicht mit dem Nukleinsäuredoppelstrang reagieren, und so markierte Einzelstrangabschnitte keine Doppelstränge mit einem komplementären Strang mehr ausbilden.

Ein großer Vorteil ist außerdem, dass sämtliche ungeschützte Einzelstrang-Nukleinsäuren durch [OsO₄(bipy)] angegriffen werden und anschließend keine Doppelstränge mehr ausbilden. Hierdurch wird die unspezifische Anlagerung von nicht komplementären Nukleinsäuresträngen an die Sondenstränge erschwert. Die Erkennung der Targetstränge in einem Überschuss an nicht-komplementären Nukleinsäuresträngen wird daher erleichtert.

Diese Vorteile zusammen können im Falle von zu detektierender mRNA die Notwendigkeit einer Vervielfältigung mittels PCR überflüssig machen, da die Nachweis-Empfindlichkeit des erfindungsgemäßen Verfahrens auch für sehr kleine RNA-Mengen ausreichend ist.

Im Schnitt kann man bei einem PCR-Produkt mit 50 bis 200 Thyminbasen pro Strang rechnen. mRNA kann über 500 Uracilbasen enthalten. Dies führt zu einem mehr als hundertfach verstärkten Signal verglichen mit herkömmlichen kovalent gebundenen Redoxmarkern wie z.B. Ferrocen, weil pro Targetstrang über 100 Elektronen umgesetzt werden.

Eine solche hochempfindliche, selektive und direkte Bestimmung von mRNA kommt ohne einen für gewöhnlich vorangehenden PCR-Schritt aus und ermöglicht so eine schnelle und einfache Analyse der Genexpression von Zellen.

Das erfindungsgemäße Verfahren hat darüber hinaus einen wesentlichen wirtschaftlichen Nutzen, da die Analytik von DNA und RNA erheblich vereinfacht wird. Es kommen elektrochemische DNA-Chips zum Einsatz, welche im Verlaufe der elektroanalytischen Methoden (Chronopotentiometrie, Amperometrie, Coulometrie, Voltammetrie) direkt auslesbare digitale Analysendaten liefern, und es sind keine teuren, speziell markierten Oligonukleotide mehr nötig. Im Falle der Genexpressionsanalysen könnte im besten Falle die teure und langwierige PCR-Vervielfältigung entfallen.

Insbesondere eröffnen sich drei wirtschaftlich interessante Anwendungen des erfindungsgemäßen Prinzips:
- Kostengünstige Herstellung von markierten Nukleinsäuresträngen zur Verwendung als elektrochemisch aktive Reportersonden, welche komplementäre Abschnitte zu Abschnitten auf den Targetsträngen enthalten und diese molekular erkennen sowie daran anschließend eine Oligothyminsequenz, welche mit [OsO₄(bipy)] oder [OsO₄(py)₂] reagiert.
- Schnelle, einfache, kostengünstige elektrochemische Analytik von mRNA-Strängen aus der Genexpression ohne vorherige PCR;
- Schnelle, einfache, kostengünstige elektrochemische Detektierung von PCR-Produkten.

Die vorliegende Erfindung betrifft somit auch Verfahren zur Genexpressionsanalyse und zum Nachweis von PCR-Produkten, d.h. die Verwendung der oben genannten Verfahren zur Genexpressionsanalyse und zum Nachweis von PCR-Produkten.

Ferner sind Gegenstand der Erfindung Kits zur Durchführung eines Verfahrens zur Genexpressionsanalyse und zum Nachweis von PCR-Produkten, umfassend ein oder mehrere Nukleinsäuren (Schutzstränge A), Redoxmarker [OsO₄(bipy)] oder [OsO₄(py)₂] und Elektroden mit auf ihrer Oberfläche immobilisierten Sonden, wobei die Nukleinsäuresequenzen der Schutzstränge und Sonden komplementär zu den nachzuweisenden Targetsträngen und kürzer als dieselben sind und die Nukleinsäuresequenzen der Schutzstränge auch eine oder mehrere Fehlpaarungen aufweisen können.

Die Erfindung wird nachfolgend auch anhand von Beispielen erläutert, wobei die Erfindung keineswegs auf diese konkreten Ausführungsbeispiele beschränkt ist.

### Beispiele

### Beispiel 1:

### Markierung eines PCR-Produktes mit [OsO4(bipy)] und elektrochemische Analyse.

Die Vorgehensweise zur Bestimmung von PCR-Produkten entsprach Beispiel 2, wobei die PCR-Produkte die gesuchte Targetsequenz enthalten.

Vor der Markierung wurde die PCR in der Reaktionsmischung mittels Protease abgebrochen. Anschließend wurde zur Denaturierung aller Doppelstränge nochmals auf 95°C aufgeheizt. Dann wurde der Schutzstrang im großen Überschuss (verglichen mit den PCR-Produkten) zugesetzt. Anschließend wurde der Schutzstrang thermisch stringent an die PCR-Produkte angelagert, auf 35°C abgekühlt, und es wurden 1 mM [OsO₄(bipy)] zugesetzt.

Nach Ablauf der Reaktion konnte das überschüssige [OsO₄(bipy)] durch Dialyse abgetrennt werden. Zum Schluss erfolgte Hybridisierung mit an Gold immobilisierten Sondensträngen unter Verdrängung der Schutzstränge und die elektrochemische Detektierung mittels Square-wave Voltammetrie.

### Beispiel 2:

### Markierung eines Targetstranges mit [OsO₄(bipy)] und elektrochemische Analyse.

Es wurden äquimolare Mengen Osmiumtetroxid (dieses ist als 2%ige Lösung in Wasser z.B. bei der Firma Fluka erhältlich) und 2,2'-Bipyridyl zu einer 10 mM Lösung des Komplexes [OsO₄(bipy)] zusammengegeben. Diese lässt sich bei -18°C lagern.

Nachdem je 16,5 µL (500 pmol) des Schutzstranges mit der Sequenz 5'-CGC GGA TAA CAC AGC CAC GC-3' (alle Oligos von der Firma Operon, Köln) und des Targetstranges mit der Sequenz 5'-TTT TTA GGT GAC TGT GTT ATC CGC A-3' für zwei Stunden bei Raumtemperatur zusammengegeben wurden, wurden 15 µl des Komplexes und 12 µl 10 mM Tris(hydroxymethyl)-aminomethan (Tris) + 0,5 M Na₂SO₄ pH 7,5 dazugegeben. Das Ganze wurde dann nach kurzem Schütteln 22. Stunden bei Raumtemperatur reagieren gelassen.

Zum Abbruch der Reaktion und zur Abtrennung von restlichem [OsO₄(bipy)] wurde eine 19-stündige Dialyse in etwa 50 ml 10 mM Trispuffer + 0,5 M Na₂SO₄ pH 7,5 bei 10°C mit Slide-A-Lyzer MINI Dialysis Units von der Firma Rockford (IL, USA) mit einem MWCO (Molekulargewicht-Durchlass) von 3500 kDa durchgeführt.

Zur Herstellung der Sonde wurden zunächst die Elektroden vorbehandelt. Die Goldscheibenelektrode wurde mit Aluminiumoxidpulver poliert. Die Golddrahtelektrode wurde durch einen 0,65 A Wechselstrom an der Luft geglüht. Dann erfolgte an beiden Elektroden eine elektrochemische Vorbehandlung, bei der 25mal ein Cyclovoltammogramm zwischen -0,2 und +1,85 V gegen eine Ag/AgCl (3 M KCl)-Referenzelektrode in 0,5 M Schwefelsäure durchlaufen wurde. Daraufhin wurden die Elektroden erst mit Wasser und dann mit Ethanol abgespült. Nach dem Trocknen wurde ein Tropfen (16.5 µL) der Sondenstranglösung (enthält 500 pmol Sondenstrang) auf die Goldoberfläche bzw. auf den Plastiksteg der Drahtelektrode gegeben. Der Sondenstrang hat die Sequenz 5'-TGC GGA TAA CAC AGT CAC CT-3' und ist mit einen CH₃-(CH₂)₂-S-S-(CH₂)₃-Disulfidlinker am 3'-Ende verbunden, über den die Anbindung an die Goldoberfläche erfolgt. Dann wurde die Elektrode über Nacht in gesättigter Wasserdampfatmosphäre bei 5°C stehen gelassen. Als nächstes wurde sie erst mit 0,25 M Phosphatpuffer pH 7,0 dann mit Wasser abgespült und zur Nachbelegung für eine Stunde in eine 1 mM wässrige Lösung von 6-Mercapto-1-Mexanol (MCH) gegeben (T.M. Herne, M.J. Tarlov, J. Am. Chem. Soc. 1997, 119, 8916-8920). Schließlich wurde sie mit Ethanol und Wasser abgespült.

Zur Hybridisierung von Sonden- und Targetstrang wurde zunächst die Lösung mit dem Doppelstrang aus markiertem Targetstrang und Schutzstrang in einem 10-ml-Becherglas auf eine Konzentration von etwa 160 nM mit 10 mM Trispuffer + 0,5 M Na₂SO₄ pH 7,5 verdünnt. Dann wurde diese Lösung 1 Minute lang im Wasserbad auf 80°C erhitzt, um den Doppelstrang aufzutrennen und die folgende Hybridisierung mit dem Sondenstrang zu erleichtern.

Daraufhin ließ man die Lösung auf eine Temperatur von 35°C abkühlen. Diese wurde mit einem Wasserbad eingestellt. Bei der Drahtelektrode wurde in einer 3°C kalten Lösung gearbeitet (hier wurde die Temperatur dann über die Heizung des Drahtes eingestellt).

Schließlich wurde die Hybridisierung eingeleitet, indem die Elektrode mit dem immobilisierten Sondenstrang in die Targetstranglösung eingetaucht wurde. Nach dem Ablauf einer bestimmten Zeit, vorzugsweise 15 Minuten, wurde dann die Elektrode mitsamt dem Doppelstrang in die Messzelle überführt.

Die Messungen wurden bei Raumtemperatur in einer Messzelle mit 20 ml 10 mM Trispuffer + 0,5 M Na₂SO₄ pH 7.5 durchgeführt.

Vor jeder weiteren Messung wurde der Doppelstrang in 50°C warmen Wasser für 30 Sekunden dehybridisiert.

Für die voltammetrischen Messungen (Square Wave Voltammetrie) wurde ein AUTOLAB^{®} der Firma Eco Chemie (Utrecht, NL) benutzt, das mit einem Potentiostaten des Typs PSTAT 10 ausgerüstet war. Gesteuert wurden die Messungen mit der Software GPES 3. Dabei betrug die Pulsamplitude 40 mV und die Frequenz 200 Hz bei einer Potentialstufe von 2 mV.

Es wurde eine 3-Elektrodenanordnung mit Ag/AgCl-Elektrode (3 M KCl) als Referenz- und einer Glaskohleelektrode als Gegenelektrode benutzt.

Als Arbeitselektrode kamen eine Goldscheibenelektrode (Durchmesser 3 mm) und eine heizbare Golddrahtelektrode zum Einsatz (Länge des Drahtes 4 mm bei einem Durchmesser von 25 µm). Glaskohle-, Goldscheiben- und Ag/AgCl-Elektrode waren von der Firma Metrohm. Die Golddrahtelektrode wurde selbst gebaut (G.-U. Flechsig, J. Peter, G. Hartwich, J. Wang, P. Gründler, Langmuir 2005, 21, 7848-7853).

Zur Beheizung der Drahtelektrode wurde ein 100 kHz Wechselstrom benötigt. Dieser wurde mittels einer Stromquelle FPS 15A DC von VOLTCRAFT^{®}, eines Funktionsgenerators MXG-9802 von VOLTCRAFT^{®} und eines Verstärkers CA2100 (Concord Car Audio, Woodbury, NY, USA) erzeugt. Am Ende der Anordnung befand sich ein Hochfrequenztransformator. Mit Hilfe eines Multimeters VOLTCRAFT^{®} M-4660A wurden die Heizströme abgelesen.

Die Beziehung zwischen Temperatur der Golddrahtelektrode und Heizstrom wurde in einem anderen Experiment bestimmt. Dabei wurden die Drahtelektrode und eine Gegenelektrode, in diesem Fall eine aus Glaskohle in eine Messzelle mit 50 mM Kaliumhexacyanoferrat (II) und 50 mM Kaliumhexacyanoferrat (III) in 0,1 M Kaliumchlorid gegeben. Die Temperatur dieser Lösung betrug 3°C. Nun wurde die Drahtelektrode beheizt und die Potentialdifferenz zwischen beiden Elektroden (potentiometrisch) gemessen. Mit Kenntnis des Temperaturkoeffizienten (β = 1.6 mV/K) ließ sich eine Kalibrierkurve ableiten, aus der hervorgeht, bei welchem Strom welche Temperatur erzeugt wird (T. Zerihun, P.Gründler, J. Electroanal. Chem. 1996, 415, 85-88; Zerihun, P.Gründler, J. Electroanal. Chem. 1996, 404, 243-248).

Die Signalhöhe des Peakstromes hängt von Hybridisierungsdauer, Hybridisierungstemperatur und Konzentration des Targetstranges ab. Eine weitere entscheidende Rolle spielt die Anzahl der markierten Pyrimidinbasen Thymin und Cytosin. In diesem Fall wurden 5 Thyminbasen markiert.

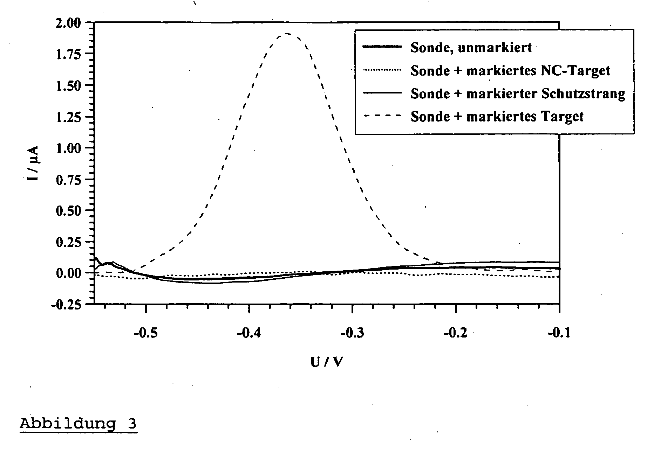

In Abbildung 3 sind die Voltammogramme von Sondenstrang, Sondenstrang und markiertem nicht komplementären Targetstrang (NC-Target) (t_{H} = 15 min, T = 23 °C, C_{NC-Target} = 163,4 nM), Sondenstrang und markiertem Schutzstrang (t_{H} = 20 min, T = 40°C, C_{Target} = 163,4 nM) und Sondenstrang und markiertem Targetstrang (t_{H} = 15 min, T = 23 °C, C_{Target} = 163, 4 nM) dargestellt. Messungen in 10 mM Trispuffer + 0,5 M Na₂SO₄ (pH = 7,5). Messungen mit der Goldscheibenelektrode (Durchmesser 3 mm). Während der Hybridisierung wurde die Lösung gerührt.

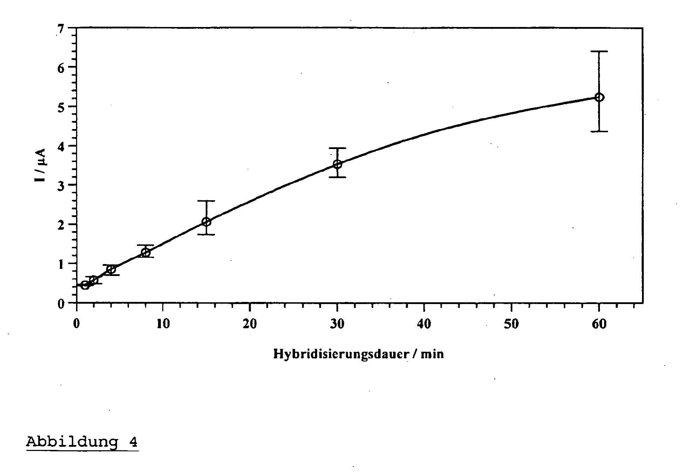

Abbildung 4 zeigt die Abhängigkeit der Peakhöhe von der Hybridisierungsdauer bei 23°C. Messungen in 10 mM Trispuffer + 0,5 M Na₂SO₄ (pH = 7,5). Targetstrangkonzentration c = 163,4 nM. Messungen mit der Goldscheibenelektrode (Durchmesser 3 mm). Während der Hybridisierung wurde die Lösung gerührt. Es sind die Mittelwerte aus drei Messungen und die maximalen Abweichungen vom Mittelwert aufgetragen.

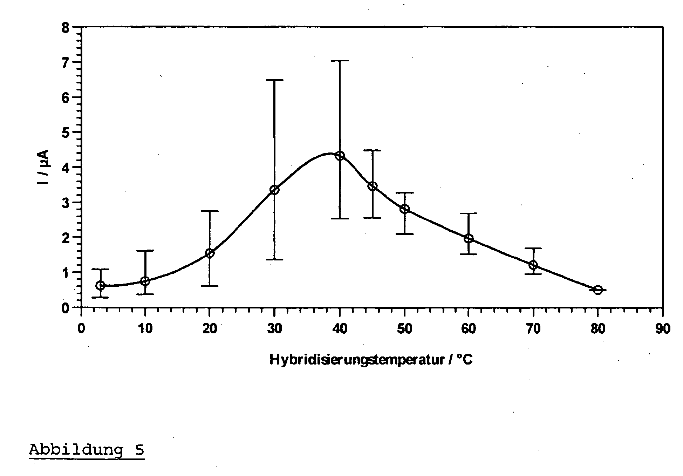

Abbildung 5 beschreibt die Abhängigkeit der Peakhöhe von der Temperatur. Messungen in 10 mM Trispuffer + 0,5 M Na₂SO₄ (pH = 7,5). Targetstrangkonzentration 163,4 nM, Hybridisierungsdauer 15 min. Messungen mit der Goldscheibenelektrode (Durchmesser 3 mm). Während der Hybridisierung wurde die Lösung gerührt. Es sind die Mittelwerte aus 3 Messungen und die maximalen Abweichungen vom Mittelwert aufgetragen.

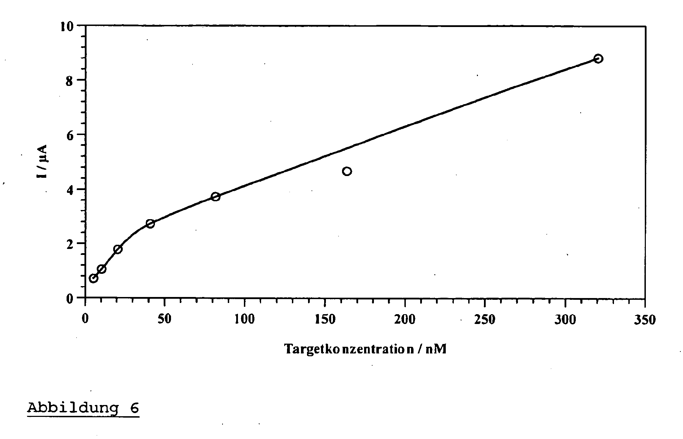

Abbildung 6 zeigt die Abhängigkeit der Peakhöhe von der Konzentration des Targetstranges. Messungen in 10 mM Trispuffer + 0,5 M Na₂SO₄ (pH = 7,5). Temperatur T = 40°C, Hybridisierungsdauer t_{H} = 15 min. Messungen mit der Goldscheibenelektrode (Durchmesser 3 mm). Während der Hybridisierung wurde die Lösung gerührt.

| | |
|---|---|
| Targetstrang: | 5'-TTT TTA GGT GAC TGT GTT ATC CGC A-3' |
| Schutzstrang: | 5'-CGC GGA TAA CAC AGC CAC GC-3' |
| Sondenstrang: | 5'-TGC GGA TAA CAC AGT CAC CT-3' mit einen CH₃-(CH₂)₂-S-S-(CH₂)₃-Disulfidlinker am 3'-Ende |

### Beispiel 3:

### Herstellung und Verwendung von Reportersträngen, die mit [OsO4(bipy)] markiert sind.

Die Vorgehensweise entsprach Beispiel 2, nur dass anstelle des Targetstranges der Reporterstrang markiert wurde, und zum Nachweis der Targetsequenz die Targetstränge mit den markierten Reportersträngen und den immobilisierten Sondensträngen hybridisiert wurden.

Die Reporterstränge enthielten außer der komplementären Sequenz eine Vielzahl von Thyminbasen an einem oder an beiden Enden. Die Reporterstränge wurden mit dem Schutzstrang hybridisiert, anschließend mit 1 mM [OsO₄(bipy)] versetzt, und dann wurde die Lösung durch Dialyse vom überschüssigen [OsO₄(bipy)] gereinigt. Die so erhaltene Lösung wurde zur Targetlösung zugesetzt, und anschließend wurden die Targetstränge mit den an der Goldelektrode immobilisierten Sondensträngen hybridisiert.

Zum Schluss erfolgte die elektrochemische Analyse durch Square-wave Voltammetrie. Auf diese Weise kam man sehr preisgünstig zu mehrfach markierten Reportersträngen, welche sehr große reversible elektrochemische Signale liefern.

### Markierung eines Reporterstranges mit [OsO4(bipy)] und elektrochemische Analyse.

Es wurden äquimolare Mengen Osmiumtetroxid (dieses ist als 2%ige Lösung in Wasser z.B. bei der Firma Fluka erhältlich) und 2,2'-Bipyridyl zu einer 10 mM Lösung des Komplexes [OsO₄(bipy)] zusammengegeben. Diese lässt sich bei -18 °C lagern.

Nachdem je 16,5 µl (500 pmol) des Schutzstranges mit der Sequenz 5'-CGG AGG TAC GGT AAC CGG-3' (alle Oligos von der Firma Operon, Köln) und des Reporterstranges mit der Sequenz 5'-TTT TTG CAG TTA CCG TAC CTC GA-3' für zwei Stunden bei Raumtemperatur zusammen gegeben wurden, wurden 15 µl des Komplexes und 12 µl 10 mM Tris(hydroxymethyl)-aminomethan (Tris) + 0,5 M Na₂SO₄ pH 7,5 dazugegeben. Das Ganze wurde dann nach kurzem Schütteln 22 Stunden bei Raumtemperatur reagieren gelassen.

Zum Abbruch der Reaktion und zur Abtrennung von restlichem [OsO₄(bipy)] wurde eine 19-stündige Dialyse in etwa 50 ml 10 mM Trispuffer + 0,5 M Na₂SO₄ pH 7,5 bei 10°C mit Slide-A-Lyzer MINI Dialysis Units von der Firma Rockford (IL, USA) mit einem MWCO (Molekulargewicht-Durchlass) von 3500 kDa durchgeführt.

Zur Herstellung der Sonde wurden zunächst die Elektroden vorbehandelt. Die Goldscheibenelektrode wurde mit Aluminiumoxidpulver poliert. Die Golddrahtelektrode wurde durch einen 0,65 A Wechselstrom an der Luft geglüht. Dann erfolgte an beiden Elektroden eine elektrochemische Vorbehandlung, bei der 25mal ein Cyclovoltammogramm zwischen -0,2 und +1,85 V gegen eine Ag/AgCl (3 M KCl)-Referenzelektrode in 0,5 M Schwefelsäure durchlaufen wurde. Daraufhin wurden die Elektroden erst mit Wasser und dann mit Ethanol abgespült. Nach dem Trocknen wurde ein Tropfen (16,5 µl) der Sondenstranglösung (enthielt 500 pmol Sondenstrang) auf die Goldoberfläche bzw. auf den Plastiksteg der Drahtelektrode gegeben. Der Sondenstrang hatte die Sequenz 5'-TGC GGA TAA CAC AGT CAC CT-3' und war mit einen CH₃-(CH₂)₂-S-S-(CH₂)₃-Disulfid-linker am 3'-Ende verbunden, über den die Anbindung an die Goldoberfläche erfolgte. Dann wurde die Elektrode über Nacht in gesättigter Wasserdampfatmosphäre bei 5°C stehen gelassen. Als nächstes wurde sie erst mit 0,25 M Phosphatpuffer (pH 7,0), dann mit Wasser abgespült und zur Nachbelegung für eine Stunde in eine 1 mM wässrige Lösung von 6-Mercapto-1-hexanol (MCH) gegeben (T.M. Herne, M.J. Tarlov, J. Am. Chem. Soc. 1997, 119, 8916-8920). Schließlich wurde sie mit Ethanol und Wasser abgespült.

Zur Hybridisierung von Reporter- und Targetstrang (Sequenz: 5'- AGG TGA CTG TGT TAT CCG CAC CCC CCT CGA GGT ACG GTA ACT GC-3') wurde zunächst die Lösung mit dem Doppelstrang aus markiertem Reporterstrang und Schutzstrang in ein 10-ml-Becherglas mit der Lösung einer gewünschten Konzentration (z.B. 163,4 nM) des Targetstranges in 10 mM Trispuffer + 0,5 M Na₂SO₄ pH 7,5 gegeben. Dann wurde diese Lösung 1 Minute lang im Wasserbad auf 80°C erhitzt, um den Doppelstrang aufzutrennen und die folgende Hybridisierung mit dem Targetstrang zu erleichtern.

Nach dem Einstellen einer gewünschten Temperatur (abhängig vom Schmelzpunkt des Doppelstranges) des Wasserbades wurde die Elektrode mitsamt dem immobilisierten Sondenstrang (bei Messungen mit dem beheizten Draht 3°C; hier wurde die Temperatur dann über die Heizung des Drahtes eingestellt) in die Lösung mit dem über den Reporterstrang markierten Targetstrang eingetaucht. Nach Ablauf von 15 Minuten wurde dann die Elektrode mitsamt dem Doppelstrang in die Messzelle überführt.

Die Messungen wurden bei Raumtemperatur in einer Messzelle mit 20 ml 10 mM Trispuffer + 0,5 M Na₂SO₄ pH 7,5 durchgeführt.

Vor jeder weiteren Messung wurde der Doppelstrang in 50°C warmen Wasser für 30 Sekunden dehybridisiert.

Für die voltammetrischen Messungen (Square Wave Voltammetrie) wurde ein AUTOLAB^{®} der Firma Eco Chemie (Utrecht, NL) benutzt, das mit einem Potentiostaten des Typs PSTAT 10 ausgerüstet war. Gesteuert wurden die Messungen mit der Software GPES 3. Dabei betrug die Pulsamplitude 40 mV und die Frequenz 200 Hz bei einer Potentialstufe von 2 mV.

Es wurde eine 3-Elektrodenanordnung mit Ag/AgCl-Elektrode (3 M KCl) als Referenz- und einer Glaskohleelektrode als Gegenelektrode benutzt.

Als Arbeitselektrode kamen eine Goldscheibenelektrode (Durchmesser 3 mm) und eine heizbare Golddrahtelektrode zum Einsatz (Länge des Drahtes 4 mm bei einem Durchmesser von 25 µm). Glaskohle-, Goldscheiben- und Ag/AgCl-Elektrode waren von der Firma Metrohm. Die Golddrahtelektrode wurde selbst gebaut (G.-U. Flechsig, J. Peter, G. Hartwich, J. Wang, P. Gründler, Langmuir 2005, 21, 7848-7853).

Zur Beheizung der Drahtelektrode wurde ein 100 kHz Wechselstrom benötigt. Dieser wurde mittels einer Stromquelle FPS 15A DC von VOLTCRAFT^{®}, eines Funktionsgenerators MXG-9802 von VOLTCRAFT^{®} und eines Verstärkers CA2100 (Concord Car Audio, Woodbury, NY, USA) erzeugt. Am Ende der Anordnung befand sich ein Hochfrequenztransformator. Mit Hilfe eines Multimeters VOLTCRAFT^{®} M-4660A wurden die Heizströme abgelesen.

Die Beziehung zwischen Temperatur der Golddrahtelektrode und Heizstrom wurde in einem anderen Experiment bestimmt. Dabei wurden die Drahtelektrode und eine Gegenelektrode, in diesem Fall eine aus Glaskohle in eine Messzelle mit 50 mM Kaliumhexacyanoferrat (II) und 50 mM Kaliumhexacyanoferrat (III) in 0,1 M Kaliumchlorid gegeben. Die Temperatur dieser Lösung betrug 3°C. Nun wurde die Drahtelektrode beheizt und die Potentialdifferenz zwischen beiden Elektroden (potentiometrisch) gemessen. Mit Kenntnis des Temperaturkoeffizienten (β = 1,6 mV/K) ließ sich eine Kalibrierkurve ableiten, aus der hervorging, bei welchem Strom welche Temperatur erzeugt wurde (T. Zerihun, P.Gründler, J. Electroanal. Chem. 1996, 415, 85-88; T. Zerihun, P.Gründler, J. Electroanal. Chem. 1996, 404, 243-248).

| | |
|---|---|
| Reporterstrang: | 5'-TTT TTG CAG TTA CCG TAC CTC GA-3' |
| Targetstrang: | 5'- AGG TGA CTG TGT TAT CCG CAC CCC CCT CGA GGT ACG GTA ACT GC-3' |
| Schutzstrang: | 5'-CGG AGG TAC GGT AAC CGG-3' |
| Sondenstrang: | 5'-TGC GGA TAA CAC AGT CAC CT-3' mit einen CH₃-(CH₂)₂-S-S-(CH₂)₃-Disulfidlinker am 3'-Ende |

### Beispiel 4:

### Untersuchung der Genexpression durch Markierung der mRNA-Kopien mit [OsO4(bipy)].

Das Verfahren lief analog zu Beispiel 2, wobei als Targetstränge die mRNA auftrat.

Die Nukleinsäureextrakte einer Zell- oder Gewebeprobe wurden mit den Schutzsträngen hybridisiert, welche einem Sequenzabschnitt des gesuchten exprimierten Gens entsprachen. Dann wurde 1 mM [OsO₄(bipy)] zugesetzt. Der größte Teil der Nukleinsäurestränge wurde dabei mit Osmium modifiziert und war nicht mehr zur Bildung von Doppelsträngen in der Lage. Nur die geschützten Abschnitte der mRNA-Kopien konnten anschließend mit den an Gold immobilisierten Sondensträngen unter Verdrängung der Schutzstränge hybridisieren. Dadurch wurden einerseits eine hohe Selektivität und andererseits eine sehr hohe Empfindlichkeit bei der anschließenden elektrochemischen Analyse durch Chronopotentiometrie erreicht. Eine Vervielfältigung.der mRNA-Kopien durch RT-PCR und PCR konnte daher entfallen.

### Beispiel 5:

### Elektrochemische Analyse der Osmium-markierten Nukleinsäuren direkt im Anschluss an die Markierungsreaktion ohne vorherige Entf-ernung des überschüssigen gelösten [OsO₄(bipy)].

Das Verfahren läuft analog zu Beispiel 2 ab, allerdings wird nach der Markierungsreaktion keine Dialyse durchgeführt, so dass das überschüssige Markierungsreagenz in der Probelösung bleibt. Diese Lösung bringt man zur Hybridisierung mit der Sondenstrangmodifizierten Arbeitselektrode in Kontakt.

Durch Anwendung der Chronocoulometrie (A. B. Steel, T. M. Herne, M. J. Tarlov, Anal. Chem. 1998, 70, 4670-4677) konnte man zwischen den elektrochemischen Signalen der immobilisierten Osmiumverbindungen und denen der gelösten [OsO₄(bipy)]-Moleküle unterscheiden.

Dazu wird ein Potentialsprungexperiment durchgeführt. Das Potential wird von -0,2 auf -0,4 V geändert. Dieser Zeitpunkt wird gleich Null gesetzt und im Folgenden die gemessene Ladung gegen sqrt(t) aufgetragen. Die chronocoulometrischen Schnittpunkte der verlängerten linearen Abschnitte mit der y-Achse (bei t=0) zeigen die Ladungen verursacht durch kapazitive Ströme und faradaysche Ströme immobilisierter Substanzen, hier durch Umsatz der immobilisierten und markierten Nukleinsäuren an.

### Vergleichsbeispiel (nicht Gegenstand der Erfindung)

### Markierung und Nachweis von Nukleinsäuresträngen mit Hilfe einer Dien-Chinon-Verbindung.

Die Modifikation der Pyrimidinbasen erfolgte durch Diels-Alder-Reaktion mit der 1,3-Dienkomponente dieser organischen Verbindung. Die elektrochemische Detektion dieses Markers geschah über die reversibel redoxaktive Chinonkomponente.

Das Verfahren läuft analog zu Beispiel 2 ab, allerdings wird anstelle der Osmiumverbindung 7,8-bis(methylen)-6,9-Dihydronaphtochinon zur Markierung der Nukleinsäuren eingesetzt.

## Patentansprüche

1. Verfahren zum Nachweis von Nukleinsäuresträngen durch Markierung mit Redoxmarkern und elektrochemischer Detektierung von Hybridisierungsereignissen, **dadurch gekennzeichnet, dass** man
a) einzelsträngige Nukleinsäurestränge (Targetstränge) mit einem oder mehreren Nukleinsäuresträngen (Schutzsträngen A) hybridisiert, die kürzer als die Targetstränge sind, um partielle Doppelstrangabschnitte auszubilden, man
b) die verbliebenen Einzelstrangabschnitte der Targetstränge durch Reaktion mit Redoxmarkern markiert, die selektiv mit der Doppelbindung der Pyrimidinringe der Nukleinsäurestränge reagieren und eine elektroanalytisch nutzbare Redoxreaktion an Arbeitselektroden ermöglichen, wobei der Redoxmarker [OsO₄(bipy)] oder [OsO₄(py)₂] ist, man
c) die so markierten Nukleinsäurestränge an der Oberfläche einer Elektrode mit dort immobilisierten Sondensträngen unter Verdrängung der Schutzstränge A hybridisiert und man
d) die an den Sondensträngen hybridisierten Nukleinsäurestränge anschließend elektroanalytisch nachweist.

2. Verfahren nach Anspruch 1, **dadurch gekennzeichnet, dass** man die Verdrängung der Schutzstränge durch die Sondenstränge in Schritt c) bei einer Temperatur durchführt, die optimal für die thermisch stringente Hybridisierung von Sonden- und Targetsträngen ist.

3. Verfahren nach Anspruch 1 oder 2, **dadurch gekennzeichnet, dass** die Elektode eine geheizte Elektrode ist.

4. Verfahren nach den Ansprüchen 1 bis 3, **dadurch gekennzeichnet, dass** die Doppelstrangabschnitte aus Schutzsträngen A und Targetsträngen eine oder mehrere Fehlpaarungen aufweisen.

5. Verfahren nach den Ansprüchen 1 bis 4, **dadurch gekennzeichnet, dass** man vor Duchführung des Schrittes c) überschüssige Redoxmarkermoleküle durch Dialyse aus der Analysenlösung entfernt.

6. Verfahren nach den Ansprüchen 1 bis 4, **dadurch gekennzeichnet, dass** die Sondenstränge in Schritt c) mit einem oder mehreren Nukleinsäuresträngen (Schutzsträngen B) hybridisiert sind und man in Schritt c) die markierten Targetstränge an der Oberfläche einer Elektrode mit dort immobilisierten Sondensträngen unter Verdrängung der Schutzstränge A und Schutzstränge B hybridisiert.

7. Verfahren nach den Ansprüchen 1 bis 6, **dadurch gekennzeichnet, dass** die überschüssigen Redoxmarker nach Schritt b) in der Analysenlösung verbleiben und man die elektrochemischen Signale der an der Oberfläche der Elektrode gebundenen Redoxmarker von den Signalen der in Lösung befindlichen Redoxmarker bei der Detektierung durch geeignete elektrochemische Analyseverfahren voneinander trennt.

8. Verfahren nach Anspruch 7, **dadurch gekennzeichnet, dass** das elektrochemische Analyseverfahren Chronocoulometrie ist.

9. Verfahren nach Anspruch 7, **dadurch gekennzeichnet, dass** das elektrochemische Analyseverfahren Chronopotentiometrie ist.

10. Verfahren nach den Ansprüchen 1 bis 9 zur Genexpressionsanalyse.

11. Verfahren nach den Ansprüchen 1 bis 10 zum Nachweis von PCR-Produkten.

12. Kit zur Durchführung eines Verfahrens nach Anspruch 10 oder 11, umfassend eine oder mehrere Nukleinsäuren (Schutzstränge A), Redoxmarker und Elektroden mit auf ihrer Oberfläche immobilisierten Sonden, wobei die Nukleinsäuresequenzen der Schutzstränge und Sonden komplementär zu den nachzuweisenden Targetsträngen und kürzer als dieselben sind, und die Nukleinsäuresequenzen der Schutzstränge auch eine oder mehrere Fehlpaarungen aufweisen können, wobei der Redoxmarker [OsO₄(bipy)] oder [OsO₄(py)_{2]} ist.

## Claims

1. Method of detecting nucleic acid strands by labelling with redox markers and electrochemical detection of hybridization events, **characterized in that**
a) single-stranded nucleic acid strands (target strands) are hybridized with one or more nucleic acid strands (protective strands A), which are shorter than the target strands, in order to form partial doublestranded sections,
b) the remaining single-stranded sections of the target strands are labelled by reaction with redox markers which react selectively with the double bond of the pyrimidine rings of the nucleic acid strands and enable an redox reaction on working electrodes which can be used for electroanalysis,
c) the nucleic acid strands labelled in this way are hybridized on the surface of an electrode to probe strands immobilized thereon, the protective strands A being displaced, and
d) the nucleic acid strands hybridized whith the probe strands are then detected by electro analysis.

2. Method according to claim 1, wherein the replacement of the protective strands by the probe strands in step c) is carried out at a temperature optimal for the thermically stringent hybridization of probe and target strands.

3. Method according to claim 1 or 2, wherein the electrode is a heated electrode.

4. Method according to claims 1 to 3, wherein the double stranded segments of protective strands A and target strands comprise one or more mismatches.

5. Method according to claims 1 to 4, wherein, before carrying out step c), excess molecules of redox marker are separated from the analytical solution by dialysis.

6. Method according to claims 1 to 4, wherein the probe strands in step c) are hybridized with one or more nucleic acid strands (protection strands B), and, in step c), the labelled target strands are hybridized at the surface of an electrode with probe strands immobilized thereto, replacing the protection strands A and the protection strands B.

7. Method according to claims 1 to 6, wherein excess redox marker remains in the analytical solution after step b), and, during detection, the electrochemical signals of the redox markers bound at the surface of the electrode are distinguished from the signals of the redox markers in solution by appropriate electrochemical analysis methods.

8. Method according to claim 7, wherein the electrochemical analysis method is chronocoulometry.

9. Method according to claim 7, wherein the electrochemical analysis method is chronopotentiometry.

10. Method according to claims 1 to 9 for expression analysis of genes.

11. Method according to claims 1 to 10 for the detection of PCR products.

12. Kit for carrying out a method according to claim 10 or 11, comprising one or more nucleic acids (protection strands A), redox marker and electrodes with probes immobilized on their surface, wherein the nucleic acid sequences of the protection strands and the probes is complementary to the target strands to be detected, and shorter than those, and wherein the nucleic acid sequences of the protection strands may also comprise one or more mismatches, wherein the redox marker is [OsO₄(bipy)] or [OsO₄(py)₂].

## Revendications

1. Procédé pour la détection de brins d'acide nucléique par marquage avec des marqueurs redox et détection électrotechnique d'évènements d'hybridation, **caractérisé en ce que**
a) on fait hybrider des brins d'acide nucléique simple brin (brins cibles) avec un ou plusieurs brins d'acide nucléique (brins protecteurs A) qui sont plus courts que les brins cibles, afin de produire des segments double brin partiels,
b) on marque les segments simple brin restants des brins cibles par réaction avec des marqueurs redox qui réagissent sélectivement avec la double liaison des cycles pyrimidine des brins d'acide nucléique et permettent une réaction redox, utilisable pour l'électro-analyse, sur des électrodes de travail, le marqueur redox étant [OsO₄(bipy)] ou [OsO₄(py)₂],
c) on fait hybrider les brins d'acide nucléique ainsi marqués, à la surface d'une électrode, avec des brins sondes immobilisés sur celle-ci, avec déplacement des brins protecteurs A et
d) on détecte ensuite par électro-analyse les brins d'acide nucléique hybridés avec les brins sondes.

2. Procédé selon la revendication 1, **caractérisé en ce qu'**on effectue le déplacement des brins protecteurs par les brins sondes dans l'étape c) à une température qui est optimale pour l'hybridation thermiquement stringente des brins cibles et sondes.

3. Procédé selon la revendication 1 ou 2, **caractérisé en ce que** l'électrode est une électrode chauffée.

4. Procédé selon les revendications 1 à 3, **caractérisé en ce que** les segments double brin à base de brins protecteurs A et de brins cibles présentent un ou plusieurs mésappariements.

5. Procédé selon les revendications 1 à 4, **caractérisé en ce qu'**avant l'exécution de l'étape c) on élimine par dialyse les molécules de marqueur redox en excès de la solution d'analyse.

6. Procédé selon les revendications 1 à 4, **caractérisé en ce que** dans l'étape c) les brins sondes sont hybridés avec un ou plusieurs brins d'acide nucléique (brins protecteurs B) et dans l'étape c) on fait hybrider les brins cibles marqués, à la surface d'une électrode, avec des brins sondes immobilisés sur celle-ci, avec déplacement des brins protecteurs A et des brins protecteurs B.

7. Procédé selon les revendications 1 à 6, **caractérisé en ce que** les marqueurs redox en excès après l'étape b) restent dans la solution d'analyse et lors de la détection on sépare les uns des autres par des procédés d'analyse électrochimique appropriés les signaux électrochimiques des marqueurs redox fixés sur la surface de l'électrode d'avec les signaux des marqueurs redox se trouvant en solution.

8. Procédé selon la revendication 7, **caractérisé en ce que** le procédé d'analyse électrochimique est la chronocoulométrie.

9. Procédé selon la revendication 7, **caractérisé en ce que** le procédé d'analyse électrochimique est la chronopotentiométrie.

10. Procédé selon les revendications 1 à 9, pour l'analyse de l'expression de gènes.

11. Procédé selon les revendications 1 à 10, pour la détection de produits de PCR.

12. Nécessaire pour l'exécution d'un procédé selon la revendication 10 ou 11, comprenant un ou plusieurs acides nucléiques (brins protecteurs A), un marqueur redox et des électrodes avec des sondes immobilisées sur leur surface, les séquences d'acide nucléique des brins protecteurs et des sondes étant complémentaires des brins cibles à détecter et étant plus courts que ceux-ci, et les séquences d'acide nucléique des brins protecteurs pouvant également présenter un ou plusieurs mésappariements, le marqueur redox étant [OsO₄(bipy)] ou [OsO₄(py)₂].


### SEQUENZPROTOKOLL

<110> Universitaet Rostock
<120> Verfahren zur Markierung und Analyse von Nukleinsaeuren
<130> P 72195
<160> 7
<170> PatentIn version 3.1
<210> 1
   <211> 25
   <212> DNA
   <213> Artificial
<400> 1
   tttttaggtg actgtgttat ccgca 25
<210> 2
   <211> 20
   <212> DNA
   <213> Artificial
<400> 2
   cgcggataac acagccacgc 20
<210> 3
   <211> 20
   <212> DNA
   <213> Artificial
<400> 3
   tgcggataac acagtcacct 20
<210> 4
   <211> 23
   <212> DNA
   <213> Artificial
<400> 4
   tttttgcagt taccgtacct cga 23
<210> 5
   <211> 44
   <212> DNA
   <213> Artificial
<400> 5
   aggtgactgt gttatccgca cccccctcga ggtacggtaa ctgc 44
<210> 6
   <211> 18
   <212> DNA
   <213> Artificial
<400> 6
   cggaggtacg gtaaccgg 18
<210> 7
   <211> 20
   <212> DNA
   <213> Artificial
<400> 7
   tgcggataac acagtcacct 20